# EUROPEAN PATENT APPLICATION

(11) **EP 3 085 411 A1**
(43) Date of publication of application: **26.10.2016**
(21) Application number: 16166714.2
(22) Date of filing: 22.04.2016
(51) Int. Cl.: A61N 1/04, A61N 1/32, A61B 17/32, A61N 7/00, A61B 18/00, A61B 18/12, A61N 1/08, A61B 17/00

(54) **ELECTRONIC APPARATUS FOR RADIO FREQUENCY OR ULTRASONIC TREATMENTS**

(30) Priority: 23.04.2015 IT MI20150582
(71) Applicant: MDM Industrial S.r.l., 40010 Sala Bolognese (BO) (IT)
(72) Inventor: Montanari, Federico, 40010 Sala Bolognese BO (IT)
(74) Representative: Ciceri, Fabio

(57) **Abstract**

The present disclosure relates to an electronic apparatus (1) for emitting electromagnetic radio-frequency or ultrasonic waves, which comprises an electronic device (2) configured to emit a frequency-variable signal (S), and a handpiece (3) connected to the terminals (21) of the electronic device (2) to receive the frequency-variable signal (S), to thereby emit a radio-frequency or ultrasonic wave; the electronic device (2) comprises a control and monitoring unit (4) for scanning a range of frequencies of the frequency-variable signal (S) during a first time interval, and an electric quantity reader (5, 7) for detecting the electric current (I) and/or the voltage (V) circulating at the terminals (21), the control and monitoring unit (4) being configured to identify a maximum value for the electric current (I) and/or the voltage (V) and to associate it with a resonance frequency value, to generate the frequency-variable signal (S), during a second time interval, at the resonance frequency value.

## Description

### Field of the invention

The present disclosure relates to an electronic apparatus, particularly but not exclusively for use in radio-frequency or ultrasound cosmetic skin treatments, as defined in the preamble of claim 1.

For example, the electronic apparatus may be also used for medical treatments.

The present disclosure also relates to a method that uses such electronic apparatus.

### Discussion of the related art

At present, there exists a strong need to mitigate the effects of aging and skin damage caused by sun exposure, because these are the most common causes for the formation of light and deep wrinkles on the face and/or other parts of the human body.

Radio-frequency or ultrasound treatments are known to be conducted using a handpiece. Namely, the apparatus comprises a machine connected to the handpiece, the latter being able to emit radio-frequency or ultrasonic waves for radio-frequency treatments.

Particularly, the handpieces have a tip or a plate (also defined as an actuator), which is configured to emit radio-frequency or ultrasonic waves when in contact with the skin of an individual.

The handpieces may be of unipolar or bipolar type.

For example, a bipolar handpiece structure incorporates an actuator and has a neutral electrode and an active electrode (which may be resistive or capacitive electrodes) in a limited area. Thus, current tends to circulate in the underlying tissue, by migrating from one electrode to the other, and treating medium-depth tissues.

The machine of a known apparatus is configured to generate a frequency signal that is sent to the handpiece connected thereto. The handpiece receives the frequency signal generated by the machine and emits a radio-frequency or ultrasonic waveform at the body surface of an individual. For example, the machine generates a highfrequency signal when it is connected to an ultrasonic handpiece. In other words, the machine manages and causes the operation of the handpiece in terms of emission of a radio-frequency or ultrasonic waveform.

### Prior art problem

An electronic apparatus comprising a machine configured to control the radio-frequency or ultrasonic waveform emitted by a handpiece connected thereto is known in the art.

Prior art machines generate the radio-frequency or ultrasonic waveform as a function of preset parameters, which are based on the particular treatment to be conducted, the area to be treated and the type of handpiece in use.

Nevertheless, a wide variety of body surfaces may be treated by radio-frequency or ultrasounds. These surfaces differ by conformation, composition and structure. The body surface (e.g. the dermis) that contacts the handpiece has highly variable surface and bulk properties, even within small portions of tissues. These properties (e.g electrical properties) determine the impedance of tissue when electric current or electromagnetic waves propagate therethrough. Therefore, the radio-frequency or ultrasonic waveform is transferred from the handpiece to the tissue with a very variable efficiency, depending on the type of tissue and the surface properties thereof, which change according to the area that is being treated. Thus, during radio-frequency or ultrasound treatment, the handpiece slides over the body surface of the individual, and encounters tissue portions with highly varying properties. Nevertheless, the handpiece emits the same waveform, based on the preset parameters, throughout the treatment.

Therefore, prior art apparatus cannot accommodate the high variability of the surface properties of body tissues. This results in a less effective transfer of the waveform through the tissue or, in extreme cases, in the transmission of excessively high currents or in excessive heating of the tissue being treated.

### SUMMARY OF THE INVENTION

The present invention has the object of providing an electronic radio-frequency or ultrasonic apparatus that can obviate the aforementioned problems of the prior art.

This object is fulfilled by an electronic radio-frequency or ultrasonic apparatus and a method associated therewith, as defined in the characteristics of the annexed claims 1 and 9 respectively.

### Advantages of the invention

One embodiment provides an electronic apparatus that can generate a radio-frequency or ultrasonic waveform according to the type of handpiece that is connected thereto, the type/conformation of the tissue being treated and the type of treatment.

One embodiment provides an electronic apparatus, and a method associated therewith, that affords real-time identification of the resonance frequency of the radio-frequency or ultrasonic waveform emitted by a handpiece.

One embodiment provides an electronic apparatus and a method associated therewith, that can always generate a radio-frequency or ultrasonic waveform with a resonance frequency depending on the impedance of the tissue being treated and the handpiece.

Finally, one embodiment provides an electronic apparatus and a method associated therewith that can always ensure effective transfer of the radio-frequency or ultrasonic waveform into the tissue being treated.

### BRIEF DESCRIPTION OF THE DRAWINGS

The characteristics and advantages of the present disclosure will appear from the following detailed description of a possible practical embodiment, illustrated as a non-limiting example in the set of drawings, in which:

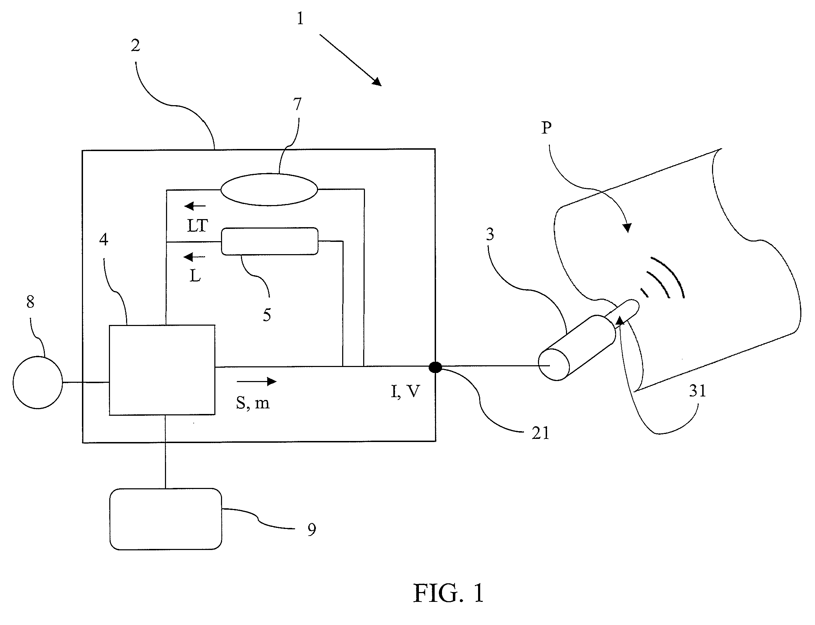

- Figure 1 shows an embodiment of an electronic apparatus of the present invention,

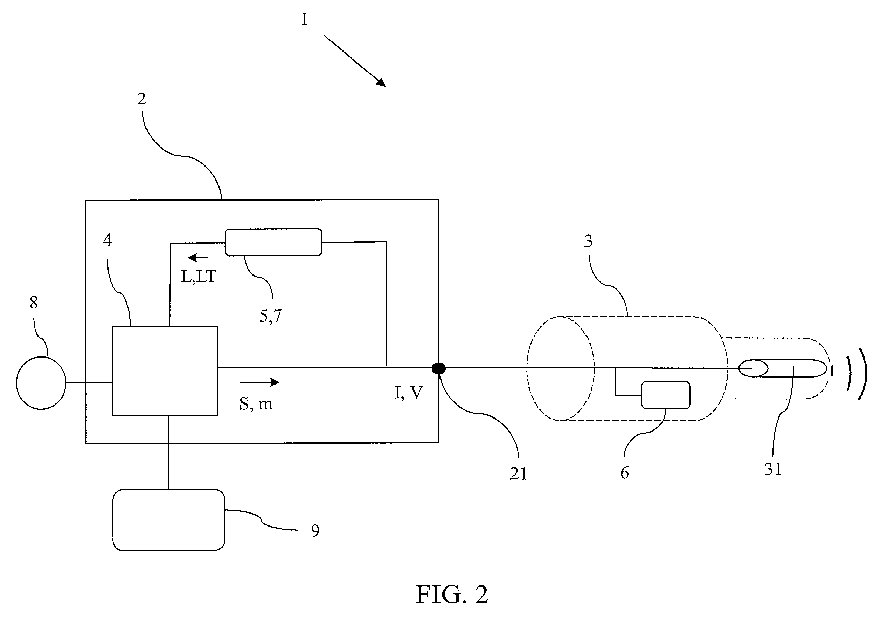

- Figure 2 shows a detailed view of a variant of the electronic apparatus of Figure 1,
- Figure 3 shows an example of the method that uses the electronic apparatus of Figure 1 or 2, according to the present invention.

### DETAILED DESCRIPTION

The apparatus of the accompanying figures shall be deemed to be schematically illustrated, not necessarily drawn to scale, and not necessarily representing the actual proportions of its parts.

Although this is not expressly shown, the individual features described with reference to each embodiment shall be intended as auxiliary and/or interchangeable with other features, as described with reference to other embodiments.

The present invention concerns an electronic apparatus 1 for emitting electromagnetic radio-frequency or ultrasonic waves.

The electronic apparatus comprises a preferably digital electronic device 2 configured to emit a frequency-variable signal S at its terminals 21.

Preferably, the frequency-variable signal S is a sine signal, although it might also assume a square or rectangular waveform.

The electronic apparatus 1 comprises a handpiece 3 that is adapted to be connected to the terminals 21 of the electronic device 2. Particularly, the connection is in the form of an electrical connection and, preferably also a mechanical connection.

The handpiece 3 is configured to receive the frequency-variable signal S, to thereby emit a radio-frequency or ultrasonic wave as a function of said frequency-variable signal S.

The handpiece 3 is also configured to contact a portion P of the body surface of an individual, to be able to transfer the radio-frequency or ultrasonic wave.

As soon as the radio-frequency or ultrasonic wave is transferred to the portion P of the body surface that contacts the handpiece 3, an electric current I circulates at the terminals 21 of the electronic device 2. The intensity of the electric current I is a function of the frequency-variable signal S and the electrical impedance of the portion P of the body surface or the handpiece 3 itself.

Preferably, the frequency-variable signal S is a carrier signal, whereby the radio-frequency or ultrasonic wave emitted by the handpiece is also a carrier wave.

In order to generate the radio-frequency or ultrasonic wave as a function of the frequency-variable signal S, the handpiece 3 comprises an actuator 31, which is known to the skilled person and will not be further described herein.

The electronic device 2 comprises a control and monitoring unit 4, which is configured to generate the frequency-variable signal S.

Particularly, the control and monitoring unit 4 is in the form of a microcontroller, having appropriate firmware, which can carry out various processing tasks, including the task of scanning a preset range of frequencies of the frequency-variable signal S, during a first preset time interval.

In other words, the control and monitoring unit 4 is configured to generate the frequency-variable signal S by scanning frequencies in a first preset range of frequencies.

According to a preferred embodiment, the handpiece 3 comprises a storage unit 6 which is configured to contain a plurality of data units stored therein, and associated with the preset range of frequencies and the first preset time interval. Thus, the handpiece 3 connected to the electronic device 2 has a characteristic range of operating frequencies for emission of radio-frequency or ultrasonic waves.

The control and monitoring unit 4 is in signal communication with the storage unit 6 and is configured to read the plurality of data units stored in the storage unit 6, to generate the frequency-variable signal S, by scanning the frequency range in the first preset time interval.

In other words, the control and monitoring unit 4 is configured to recognize the handpiece 3 connected to the electronic device 2, to thereby identify its proper operating parameters.

The electronic device 2 further comprises an electric quantity-measuring device (5, 7), such as a current reader 5 and/or a voltage reader 7, each being configured to detect the current I that circulates at the terminals 21 of the electronic device 2 and/or the voltage (V) existing at the terminals 21 of the electronic device 2, to generate a read signal L as a function of the intensity of the electric current I and/or a read signal LT as a function of the intensity of the voltage V respectively.

Preferably, the read signal L and/or the read signal LT are digital signals.

The control and monitoring unit 4 is in signal communication with the current reader 5 and/or the voltage reader 7 and is configured to sample the read signal L and/or LT during the first preset time interval, and process the read signal L and/or LT so sampled to calculate the maximum intensity value for the electric current I and/or the voltage V.

The control and monitoring unit 4 is configured to associate the maximum intensity value for the electric current I and/or the voltage V with a corresponding resonance frequency value for the frequency-variable signal S.

The resonance frequency of the circuit is generally related to the impedance of the portion P of the body surface that contacts the handpiece 3 and the impedance of the handpiece 3 itself.

For example, the resonance frequency is the frequency at which the electric current I that circulates at the terminals 21 of the electronic device 2 reaches its maximum intensity that causes resonance.

For instance, the control and monitoring unit 4 is configured to scan frequencies in the preset frequency range, and to process the read signal L to sample the measured intensity values for the electric current I as detected in the first preset time interval.

The control and monitoring unit 4 is also configured to calculate the maximum intensity value for the electric current I in the sampling range, to thereby associate such maximum value of electric current I with a corresponding frequency value of the frequency-variable signal S.

In other words, the resonance frequency value corresponds to the frequency value of the frequency-variable signal S generated by the control and monitoring unit 4 at the time in which the maximum intensity for the electric current I is detected. Of course, the resonance frequency value falls in the preset range of frequencies.

Likewise, the control and monitoring unit 4 is configured to associate an intensity value for the electric current I and/or the voltage V falling in a range of ±10% the maximum intensity value for the electric current I and/or the voltage V, with a corresponding resonance frequency value for the frequency-variable signal S.

Preferably, the control and monitoring unit 4 is configured to associate, using an algorithm, a maximum intensity value (or a value that falls in a range of ±10% the maximum intensity value) for the electric current I and/or the voltage V with a corresponding resonance frequency value for the frequency-variable signal S.

The control and monitoring unit 4 is configured to generate the frequency-variable signal S during a second preset time interval, with a frequency value that is equal to the resonance frequency.

Advantageously, the electronic apparatus 1 can emit radio-frequency or ultrasonic waves at the appropriate resonance frequency as a function of both the impedance of the surface portion P to be treated, that changes from point to point, and the impedance of the handpiece.

For example, the use of a gel interposed between the tip or plate of the handpiece 3 or the portion P of the body surface being treated causes a change in the impedance of the surface portion P to be treated and hence in the overall impedance obtained with the impedance of the handpiece.

It will be appreciated that the electronic apparatus 1 can emit radio-frequency or ultrasonic waves while adjusting the resonance frequency as a function of the impedance value of the handpiece with a given treated surface.

According to a preferred embodiment, the control and monitoring unit 4 is configured to generate an amplitude-variable modulating signal m, during a third preset time interval, with a preset frequency value, and with an amplitude value set as a function of the peak-to-peak voltage of the frequency-variable signal S.

Preferably, the frequency of the amplitude-variable modulating signal m is equal to a given frequency value (which is much lower than the frequency of the carrier) and its amplitude ranges from 90% to 0% the amplitude of the peak-to-peak voltage (Vₚₑₐₖ₋ₜₒ₋ₚₑₐₖ) of the frequency-variable carrier signal S.

More preferably, the third preset time interval coincides with the second preset time interval, to thereby obtain a superposition of the frequency-variable signal S (i.e. the carrier) and the amplitude-variable modulating signal m (i.e. the modulating signal).

In other words the amplitude-variable modulating signal m is repeated with a fixed frequency and for a preset duration in the second preset time interval, while being superimposed on the frequency-variable signal S.

By this arrangement, the frequency-variable signal S (carrier signal) will be reduced when the amplitude-variable modulating signal m (modulating signal) is also emitted.

Preferably, the actuator 31 is configured to generate the radio-frequency or ultrasonic wave as a function of the frequency-variable signal S, and of the amplitude-variable modulating signal m.

Advantageously, the control and monitoring unit 4 can generate a frequency signal composed of a carrier component (i.e. the frequency-variable signal S) and a modulating component (i.e. the amplitude-variable modulating signal).

Furthermore, the handpiece 3 can emit a frequency or ultrasonic wave having a carrier component and a modulating component for improved transfer of the electromagnetic wave into the tissue being treated.

Preferably, the control and monitoring unit 4 comprises a power network for generating frequency signals, i.e. the frequency-variable signal S and the amplitude-variable modulating signal m.

In accordance with a preferred arrangement, the electronic device 2 comprises a voltage reader 7, which is configured to detect the voltage V existing at the terminals 21 of said electronic device 2, to thereby generate a voltage reading signal LT as a function of the value of the voltage V.

Preferably, the control and monitoring unit 4 is in signal communication with the voltage reader 5 and is configured to sample the voltage read signal LT during the first preset time interval. The control and monitoring unit 4 is further configured to process the voltage read signal LT, to thereby monitor the value of the voltage V.

Advantageously, the electronic device 2 is able to constantly monitor the values of the electric current I and voltage V in the circuit, to ensure safety and proper operation of the electronic apparatus I.

According to a preferred embodiment of the present invention, the electronic device 2 comprises a power supply unit 8, which is configured to supply power to the electronic device 2 and the handpiece 3. Preferably, the power supply unit 8 is configured to supply power to the control and monitoring unit 4.

Preferably, the electronic device 2 comprises a graphical interface 9 in signal communication with the control and monitoring unit 4 to allow a user of the electronic apparatus 1 to control and monitor its operation.

For example, a user can select the type of treatment to be conducted, also according to the area of the body to be treated, to thereby optimize the operating parameters of the electronic apparatus 1.

According to a preferred embodiment, the electronic apparatus 1 comprises a plurality of bipolar or unipolar handpieces 3 for emitting radio-frequency or ultrasonic waves, which may be individually and interchangeably connected with the device 2.

Advantageously, this will provide a highly versatile electronic apparatus 1 that can emit electromagnetic radio-frequency or ultrasonic waves according in an application-specific manner.

The present disclosure also concerns a method of emitting electromagnetic radio-frequency or ultrasonic waves using an electronic apparatus 1 of the present invention.

The method includes the steps of:
a) - generating the frequency-variable signal S, by scanning a preset range of frequencies of the frequency-variable signal S during a first preset time interval, this step is preferably carried out by the control and monitoring unit 4;
b) - detecting an electric quantity I, V at the terminals 21 of the electronic device 2, to generate a read signal L, LT as a function of the intensity of the electric current i and/or the voltage V, this step is preferably carried out by the current reader 5;
c) - sampling the read signal L and/or LT during the first preset time interval, and process the read signal L and/or LT to calculate the maximum intensity value for the electric current I and/or the voltage V, this step is preferably carried out by the control and monitoring unit 4;
d) - associating the maximum intensity value for the electric current I and/or the voltage V with a corresponding resonance frequency value for the frequency-variable signal S, this step is preferably carried out by the control and monitoring unit 4;
e) generating the frequency-variable signal S during a second preset time interval, with a frequency value that is equal to said resonance frequency, this step is preferably carried out by the control and monitoring unit 4;

According to a preferred embodiment, the step c) comprises the step of sampling the read signal L and/or LT during the first preset time interval, and calculating a maximum intensity value for the electric current I and/or the voltage V.

Likewise, the step d) consists in associating an intensity value for the electric current I and/or the voltage V, such value falling in a range of ±10% the maximum intensity value for the electric current I and/or the voltage V, with a corresponding resonance frequency value for the frequency-variable signal S.

Preferably, the step d) consists in associating, using an algorithm, a maximum intensity value (or a value in the range of ±10% the maximum intensity value) for the electric current I and/or the voltage V with a corresponding resonance frequency value for the frequency-variable signal S. In other words, an algorithm may be used to calculate a resonance frequency value for the frequency-variable signal S as a function of the maximum intensity value (or a value in the range of ±10% the maximum intensity value) for the electric current I and/or the voltage V.

According to a preferred arrangement, the method comprises the step of iteratively repeating the steps a) to e) for a preset number of iterations.

According to a preferred arrangement, the method comprises the step of generating an amplitude-variable modulating signal m, during a third preset time interval, with a preset frequency value, and with an amplitude value set as a function of the peak-to-peak voltage of the frequency-variable signal S. This step being preferably carried out by the control and monitoring unit 4.

Advantageously, the method of the present invention can cyclically scan the preset range of frequencies to identify the proper resonance frequency according to the portion P of the body surface that contacts the handpiece 3. During radio-frequency or ultrasound treatment, the handpiece 3 is slid over the body surface of the individual, and encounters body regions with different electrical impedances. This will constantly ensure the most efficient transfer of radio-frequency or ultrasonic waves into the tissue being treated.

According to a preferred embodiment, the step a) is preceded by the step a0) of reading the plurality of data units stored in the storage unit 6. Such data units being associated with the preset range of frequencies and the first preset time interval. This step being preferably carried out by the control and monitoring unit 4.

An application example of the method of the present invention is described below.

### EXAMPLE

As used hereinafter, the time To is the start time.

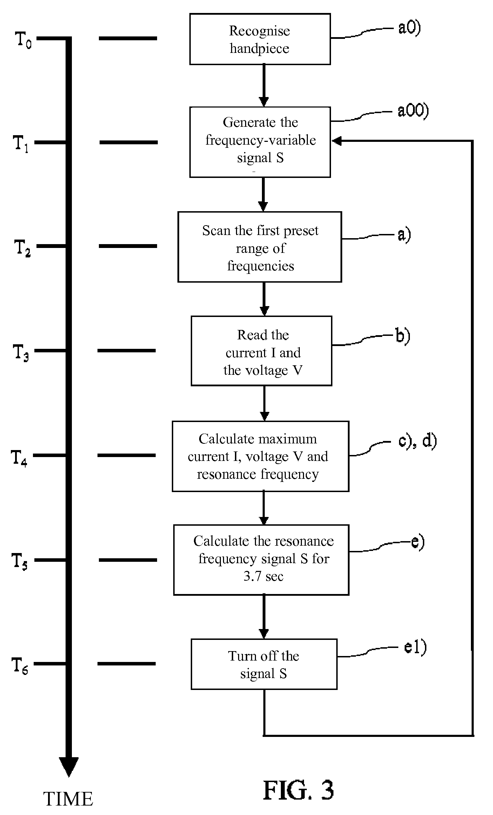

Referring to Figure 3, the steps of an implementation example of the method of the present invention are shown.

Step a0) - At the start time To the control and monitoring unit 4 reads the plurality of data units in the storage unit 6 of the handpiece 3. By doing this, it will acquire the information concerning the preset range of frequencies that is specific to the handpiece 3 and, as a result, also the duration of the first preset time interval required for scanning frequencies in the preset range of frequencies.

Step a00) - At time T₁, e.g. 50ms after time To, the control and monitoring unit generates the frequency-variable signal S.

Step a) - At time T₂, e.g. 100ms after time To, the control and monitoring unit performs scanning (or tuning) of the preset range of frequencies for the first preset time interval, e.g. 100ms.

Step b) - At time T₃, e.g. 200ms after time To, the current reader 5 and the voltage reader 7 read the values of electric current I and voltage V respectively at the terminals 21 of the electronic device 2.

Steps c) and d) - At time T₄, e.g. 300ms after time To, the control and monitoring unit 4 finds the maximum intensity for the electric current I and associates it with the corresponding resonance frequency value of the frequency-variable signal S.

Step e) - From time T₄ to time T₅, e.g. 4,000ms after time To, the control and monitoring unit 4 generates the frequency-variable signal S with a frequency value equal to the resonance frequency value. In this example, the frequency-variable signal S is emitted for a first preset time interval of 3,7 s.

If a modulating signal is also emitted in addition to the frequency-variable signal S (i.e. the carrier signal), an amplitude-variable modulating signal m, having a preset frequency, will be also generated. For example, the amplitude-variable modulating signal m is emitted every 0.5 s for a duration of 200 ms, and is superimposed on the frequency-variable signal S (carrier) for 3.7 s.

Step e1) - At time T₆, e.g. 4,100 ms after time To, the control and monitoring unit 4 stops generating the frequency-variable signal S.

The method includes a preset number of cycle, repeating all the steps from step a00).

Those skilled in the art will obviously appreciate that a number of changes and variants as described above may be made to fulfill particular requirements, without departure from the scope of the invention, as defined in the following claims.

## Claims

1. An electronic apparatus (1) for emitting electromagnetic radio-frequency or ultrasonic waves, comprising:
- an electronic device (2) configured to emit a frequency-variable signal (S) at its terminals (21),
- a handpiece (3), electrically connected to the terminals (21) of said electronic device (2) and configured to receive said frequency-variable signal (S), to emit a radio-frequency or ultrasonic wave as a function of said frequency-variable signal (S),
- a control and monitoring unit (4) in signal communication with an electric quantity-measuring device (5, 7).
**characterized in that**
- said electric quantity-measuring device (5, 7) is configured to detect a current (I) and/or a voltage (V) at the terminals (21) of said electronic device (2), to generate a read signal (L, LT) as a function of the intensity of said current (I) and/or voltage (V); when said handpiece contacts a portion (P) of the body surface of an individual,
- said control and monitoring unit (4) is configured to:
- scan a preset range of frequencies of said frequency-variable signal (S) in a first preset time interval,
- process the read signals (L, LT) generated during said first time interval, to calculate a maximum intensity value for said electric current (I) and/or said voltage (V),
- associate said maximum intensity value for said electric current (I) and/or said voltage (V) with a corresponding resonance frequency value for said frequency-variable signal (S),
- generate said frequency-variable signal (S) in a second preset time interval, with a frequency value that is equal to said resonance frequency.

2. An electronic apparatus (1) as claimed in claim 1, wherein said handpiece (3) comprises an actuator (31), configured to generate said radio-frequency or ultrasonic wave as a function of said frequency-variable signal (S), said frequency-variable signal (S) being a carrier signal.

3. An electronic apparatus (1) as claimed in claim 2, wherein
- said control and monitoring unit (4) is configured to generate an amplitude-variable modulating signal (m), in a third preset time interval, with a preset frequency value, and with an amplitude value set as a function of the peak-to-peak voltage of said frequency-variable signal (S),
- said actuator (31) is configured to generate said radio-frequency or ultrasonic wave as a function of said frequency-variable signal (S), and of said amplitude-variable modulating signal (m)

4. An electronic apparatus (1) as claimed in any of the preceding claims, wherein
- said handpiece (3) comprises a storage unit (6) which is configured to contain a plurality of data units stored therein, and associated with said preset frequency range and said preset first time interval,
- said control and monitoring unit (4) is in signal communication with said storage unit (6) and is configured to read said plurality of data units stored in said storage unit (6), to generate said frequency-variable signal (S), by scanning said frequency range in said first preset time interval.

5. An electronic apparatus (1) as claimed in any of the preceding claims, wherein
- the electric quantity-measuring device (5, 7) comprises a voltage reader (7) which is configured to detect a voltage (V) at the terminals (21) of said electronic device (2), to generate a voltage reading signal (LT) as a function of the value of said voltage (V),
- said control and monitoring unit (4) is in signal communication with said voltage reader (7) and is configured to sample said voltage reading signal (LT) in said first preset time interval, and to process said voltage reading signal (LT) to thereby monitor the value of said voltage (V),

6. An electronic apparatus (1) as claimed in any of the preceding claims 1 to 4, wherein
- said electric quantity-measuring device (5, 7) comprises a current reader (5) which is configured to detect an electric current (I) circulating at the terminals (21) of said electronic device (2), to generate a read signal (L) as a function of the intensity of said electric current (I), when said handpiece contacts a portion (P) of the body surface of an individual,
- said control and monitoring unit (4) is in signal communication with said current reader (5) and is configured to sample said current reading signal (L) in said first preset time interval, and to process said current reading signal (L) to thereby monitor the value of said current (V),

7. An electronic apparatus (1) as claimed in any of the preceding claims, wherein said control and monitoring unit (4) is configured to sample said read signal (L) in said first preset time interval.

8. An electronic apparatus (1) as claimed in any of the preceding claims , wherein said control and monitoring unit (4) comprises a microcontroller.

9. An electronic apparatus (1) as claimed in any of the preceding claims, wherein said electronic device (2) comprises a graphical interface (9) in signal communication with said control and monitoring unit (4) to allow a user of said electronic apparatus (1) to control and monitor the operation of said electronic apparatus (1).

10. A method of emitting electromagnetic radio-frequency or ultrasonic waves using an electronic apparatus (1) as claimed in any of the preceding claims, comprising the steps of:
a) generating said frequency-variable signal (S), by scanning a preset range of frequencies of said frequency-variable signal (S) during a first preset time interval,
b) detecting an electric current (I) and/or a voltage (V) at the terminals (21) of said electronic device (2), to generate a read signal (L, LT) as a function of the intensity of said electric current (I) and/or said voltage.
c) sampling said read signal (L, LT) during said first preset time interval, and process said read signal (L, LT) to calculate the maximum intensity value for said electric current (I) and/or said voltage (V),
d) associating said maximum intensity value for said electric current (I) and/or said voltage (V) with a corresponding resonance frequency value for said frequency-variable signal (S),
e) generating said frequency-variable signal (S) during a second preset time interval, with a frequency value that is equal to said resonance frequency.

11. A method as claimed in claim 10, comprising the step of iteratively repeating the steps a) to e) for a preset number of iterations.
